# EUROPEAN PATENT APPLICATION

(11) **EP 1 516 618 A1**
(43) Date of publication of application: **23.03.2005**
(21) Application number: 04021895.0
(22) Date of filing: 15.09.2004
(51) Int. Cl.: A61K 31/122, A61K 35/36, A61K 35/12

(54) **Coenzyme Q10 as antiapoptotic agent**

(30) Priority: 16.09.2003 IT MI20031767
(71) Applicant: Visufarma S.R.L., 00191 Roma (IT)
(72) Inventor: Capaccioli, Sergio Dpt. di Pat.+Onc. Sperimentali, 50134 Firenze (IT); Witort, Ewa Dipart. di Patol.+Onco. Sperimentali, 50134 Firenze (IT); Lo Russo, Domenico- Dpt. di Scienze Ortop. Ric, 50139 Firenze (IT); Papucci, Laura Dipart. di Patol.+Onc. Sperimentali, 50134 Firenze (IT); Schiavone, Nicola - Dpt. di Pat.+Onc. Sperimentali, 50134 Firenze (IT); Lapucci, Andrea- Dipat. di Pat.+Onc. Sperimentali, 50134 Firenze (IT); Donnini, Martino- Dpt. di Pat.+Onc. Sperimentali, 50134 Firenze (IT); Dini, Mario - Dipart. di Scienze Ortopediche, 50139 Firenze (IT); Pattarino, Jacopo - Dipart. di Scienze Ortopediche, 50139 Firenze (IT)
(74) Representative: Banfi, Paolo

(57) **Abstract**

The invention discloses the use of coenzyme Q10 for preventing or inhibiting apoptosis in cutaneous or adipose cells and tissues, and pharmaceutical compositions containing coenzyme Q10 for the treatment of transplanted tissues.

## Description

The present invention regards the use of coenzyme Q 10 (ubiquinone) as an antiapoptotic agent. More specifically, the invention provides the use of coenzyme Q10 for the prevention or inhibition of apoptosis in cutaneous and adipose cells and tissues. According to the invention, compositions containing coenzyme Q10 are conveniently used in plastic and reconstructive surgery, especially for lipofilling and flap graft.

### Background of the invention

Ubiquinone or coenzyme Q10 (2.3-dimethoxy-5-methyl-6-multiprenyl-1.4-benzoquinone) is a well-known component of the respiratory chain as well as an antioxidant molecule and free radical scavenger.

Previous studies demonstrated that the coenzyme Q10 is able to inhibit apoptosis in cultured keratocytes treated with the excimer laser irradiation, an apoptotic stimulus that determines free radical formation (3) and causes lowering of intracellular ATP levels. These effects have been also demonstrated in vivo (4).

More recently, it has also been shown that in response to any apoptotic stimulus ubiquinone directly inhibits disruption of the mitochondrial transmembrane electrochemical gradient as well as the opening of mitochondrial Permeability Transition Pore (mPTP) (5), whereby several molecules involved in caspase cascade activation, including Cytochrome C and APAF-1, are extruded to the cytoplasm thus provoking apoptosis.

EP 132113 8 discloses the use of coenzyme Q10 for the treatment of ocular pathologies involving apoptotic events caused by hypoxia, ischemia or lack of trophic factors.

WO01/37851 discloses the use of coenzyme Q10 for the local treatment of ophthalmologic pathologies following photoreactive therapy, refractive surgery and exposure to ultraviolet radiation.

### Disclosure of the invention

The invention is based on the finding that coenzyme Q10 is able to prevent or inhibit apoptosis in adipocytes and cutaneous cells from explanted human tissues. Increased survival of explanted or cultured tissues and cells improves the feasibility and efficacy of human tissue transplantation.

Explanted tissues and cells are subjected to several treatments prior to their reintroduction in the human body. During these treatments, a high level of cell death is observed as a consequence of apoptotic events. Cells maintained in the presence of coenzyme Q10 are less susceptible to apoptosis. In particular, the number of apoptotic events result significantly reduced in adipocytes and cutaneous cells maintained in the presence of coenzyme Q10. This result was confirmed by measuring the amount of the apoptotic indicators caspases 3 and 9 in cells from explanted tissues, after subjecting the cells to several *ex vivo* treatments aimed at their subsequent implantation. The levels of caspases were reduced by more than 50% in the presence of coenzyme Q10. The decrease of caspase 9, which is an indicator of mitochondrial apoptosis, was accompanied by an equivalent decrease of caspase 3, which is involved in both the mitochondrial and transmembrane apoptotic pathways.

Without binding the invention to a specific mechanism of action, the effects observed with ubiquinone are likely due to an activity of apoptosis prevention exerted by the enzyme Q 10 both as a free radical scavenger and through the regulation of the open/close state of the mitochondrial permeability transition pore.

Accordingly, in a first aspect the invention provides a method for preventing or inhibiting apoptosis in cells from explanted adipose or cutaneous human tissues, which comprises contacting *ex vivo* the tissues, or cells isolated therefrom, with coenzyme Q10.

In a preferred embodiment, coenzyme Q10, or a suitable composition thereof, is added to a culture of adipose or cutaneous tissues or cells; the addition can be effected at any stage of the treatment preceding cell or tissue implantation, in particular during fractionation, separation, centrifugation and freezing procedures. For these applications, coenzyme Q10 is preferably diluted in a water solution containing an amphipathic co-solvent such as polyethylene glycols and poloxamers, preferably Lutrol® .

Inhibition of apoptosis in adipocytes and cutaneous cells is important for plastic and reconstructive surgery, particularly for lipofilling and flap graft techniques. As used herein, flap graft refers to the transplantation of tissue portions that are either completely separated from donor blood vessels, and therefore have to be implanted in a highly vascularized recipient site, or that remain in contact with donor vessels (peduncle) and therefore can be implanted in a low-vascularized recipient site. In the technique known as lipofilling, the adipose tissue is collected with syringes, contacted with coenzyme Q 10 and reinoculated in the body site to be treated.

The viability of transplanted tissues can be increased by apoptosis inhibition as a consequence of ischemia and reperfusion injury reduction, thereby allowing for the transplantation of larger tissue areas.

According to a further aspect, the invention provides the use of ubiquinone Q10 for the preparation of a pharmaceutical composition for preventing or inhibiting apoptosis in transplanted tissues, especially cutaneous and adipose tissues, to increase their survival, thriving and vascularization. In this case, coenzyme Q10 is preferably administered directly to the transplantion site. Suitable pharmaceutical formulations for topical administration include solutions, injectables, creams, ointments, gel, and controlled-release transdermic delivery systems. For use in therapy, coenzyme Q10 will be formulated with pharmaceutically acceptable carriers and excipients.

### Description of the Figures

**Figure 1.** Dosage of cytoplasmic cytochrome C in adipocytes by Western Blotting.
**Figure 2.** Activity of caspases 3 and 9 in adipocytes collected both manually using a "Luer-lock" syringe and by a liposuctor. The histograms show that the activity of each caspase is reduced by nearly 50% in coenzyme Q10-treated samples compared to controls (untreated samples).
**Figure 3.** Quantitative data of caspases 3 and 9 in adipocytes obtained by Western Blotting bands analysis.
**Figure 4.** Appearance of skin grafts untreated or treated with coenzyme Q 10 and stained with *in situ* TUNEL.
**Figure 5.** Number of apoptotic cells in skin grafts untreated or treated with coenzyme Q10 and stained with *in situ* TUNEL. Values are expressed as percentage of total cells (living + dead cells).

### Materials and Methods

### Quantification of apoptotic factors (cytocrome c, caspase 3 and 9)

Adipocytes have been collected both manually using a "Luer-lock" 10 cc. syringe following Coleman's technique, and by a liposuctor with a negative pressure adjusted to maintain -680 mmHg. After the collection, half of the sample was treated with coenzyme Q10 dissolved in Lutrol (or another amphipathic molecule) at 10 µM concentration, as reported in previous works (1, 3). The other half of collected sample was treated with Lutrol (or another amphipathic molecule) alone. Both samples were then centrifuged. The interphase and cell pellet were collected, following Coleman's technique (6) and maintained in liquid Nitrogen.

Preparation of protein cell lysates (6) is a modification of the method described by Rioux (7).

Liposucted cell suspensions were centrifuged at 800 g for 10 minutes. 1 ml of lysis buffer (0.75 M NaCl, 10 mM HEPES, 1 mM EDTA, 1% Triton-X-100) containing a protease inhibitor mixture (final concentrations 1 µg/ml leupeptin, 10 µg/ml benzamidine, 1 µg/ml chymostatin, 1 µg/ml pepstatin A and 100 µg/ml PMSF) was added and the cell pellet was sonicated two times successively (20 sec, 100 W) to obtain whole cellular lysate. The lysate was further cleared by centrifugation at 10.000 g for 30 min in mini centrifuge. After removing the fat layer and the pellet of cellular debris, the protein content was quantified with Bradford reagent by spectrophotometry. Protein lysates (30 µg/lane) were separated by SDS-PAGE (10 and 15%) and analysed by Western blot analysis with specific antibodies.

Quantification of apoptotic factors was performed by densitometry.

### Isolation of adipocytes from adipose tissue

Modification of protocol described by Flower et al., Cytokine 21 (2003) 32-37.

For isolation, adipocyte tissue was quickly thawed, dissected with scissors into fine fragments in digest solution consisting of Hank's buffer, supplemented with 2 mg/ml of collagenase (Type II, Sigma) and 1.5% BSA, and incubated at 37°C for 1 hour in shaker-heater bath. The ratio of digest solution to adipose tissue was 4:1. The tissue digest was filtered through 200 µm Nylon mesh fabric in order to remove non digested tissue fragments. The adipocyte-containing layer and free oil were separated from the resulting stromovascular cell suspension by repeated centrifugation at 250 x g for 5 minutes at 4°C. All the cells retrieved were morphologically typical mature adipocytes.

### Detection of apoptotic adipocytes by Hoechst staining

Isolated adipocytes in suspension were fixed with 4% paraformaldehyde for 20 min at RT (room temperature), washed 3 times with PBS and permeabilized for 5 min with 0.25% Triton X-100 in PBS solution. Washes with PBS were repeated 3 times for 5 min each time and the cells were stained with Hoechst 33258 (Sigma) nuclear dye for 30 min at 37°C followed by washing in PBS 3 times for 5 min.

The stained cells (10⁴) were affixed to polylysine-treated glass slides by gentle cyto-centrifugation or by spreading the cellular film directly on the surface of the glass slide and mounted. Nuclear staining was examined under Nikon fluorescence microscope (20x and 40x magnification) equipped with camera. The results are expressed as the percentage of apoptotic cells (number of apoptotic cells/100 counted cells) in 10 random fields, performed by 3 independent observers.

### Results

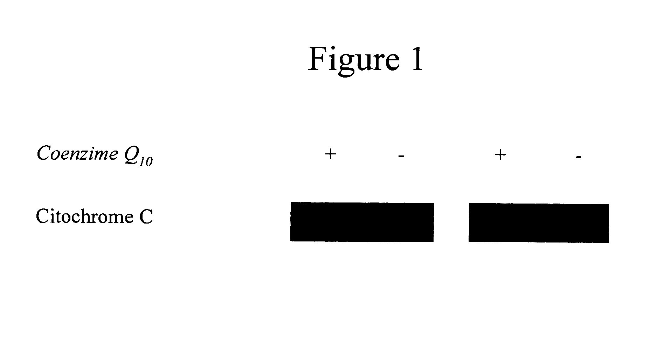

Cytochrome C is an enzyme involved in mobile electron transport and essential to energy conversion in all aerobic organisms (8). In mammalian cells, this highly conserved molecule is normally localized to the mitochondrial intermembrane space (9). Release of Cytochrome C into cytosol has been identified as an event necessary for execution of apoptosis (10). Western blot analysis of adipocyte protein lysates demonstrated that release of cytochrome C to cytoplasm (**Figure 1**) was triggered by stress associated with harvesting of adipocyte samples. Treatment of liposucted adipocytes with coenzyme Q10 significantly inhibited the translocation of cytochrome C to cytosol.

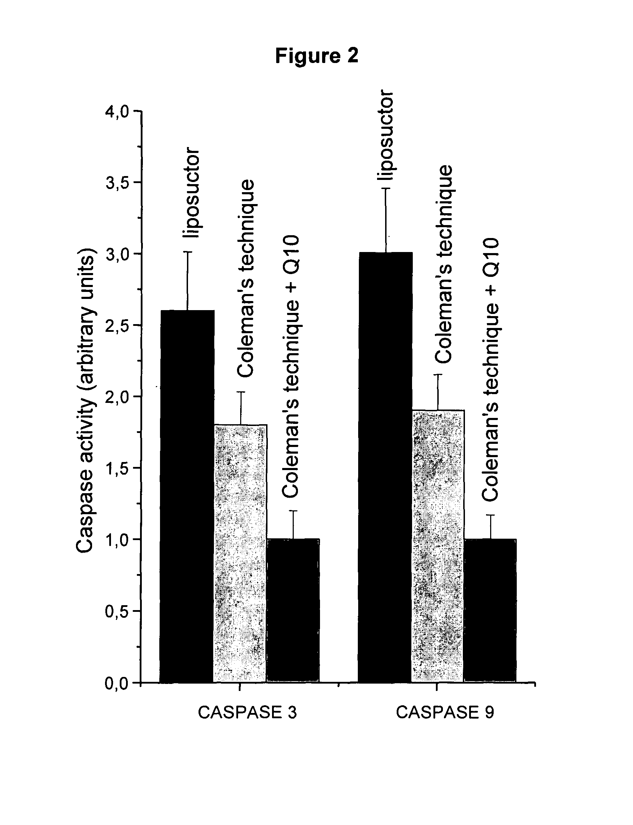

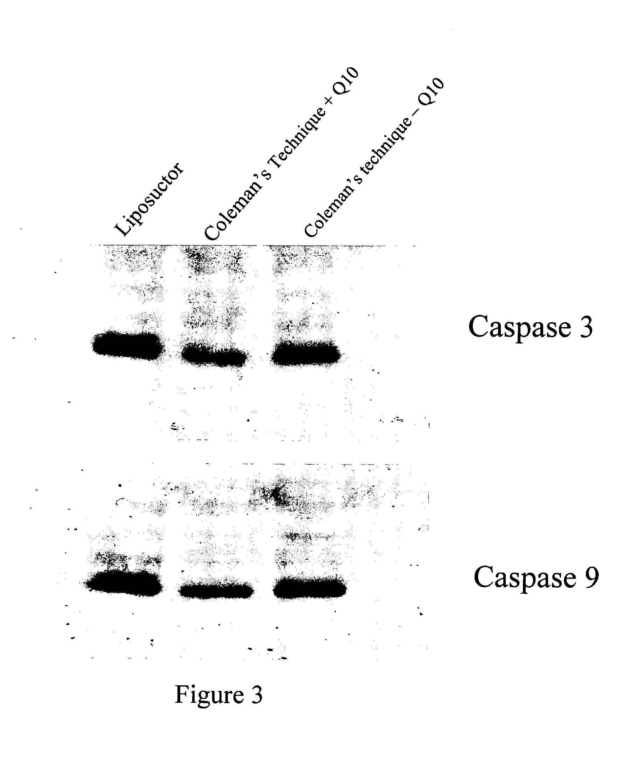

The activities of both caspases 3 and 9 evaluated by Western blotting were higher when the adipocytes were collected with the liposuctor than following Coleman's technique. The addition of coenzyme Q10 prior to centrifugation and purification dramatically reduced the activities of both caspases in adipocytes collected with Coleman's technique (**Figure 2**). Quantitative data obtained by the analysis of band intensities are shown in **Figure 3**.

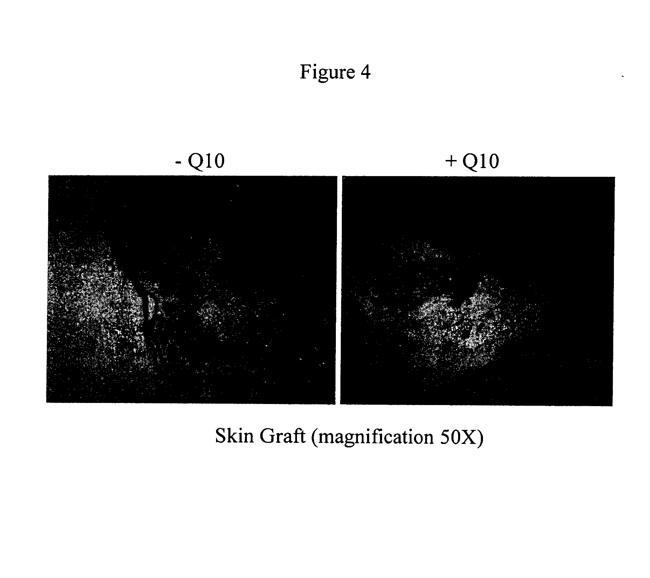

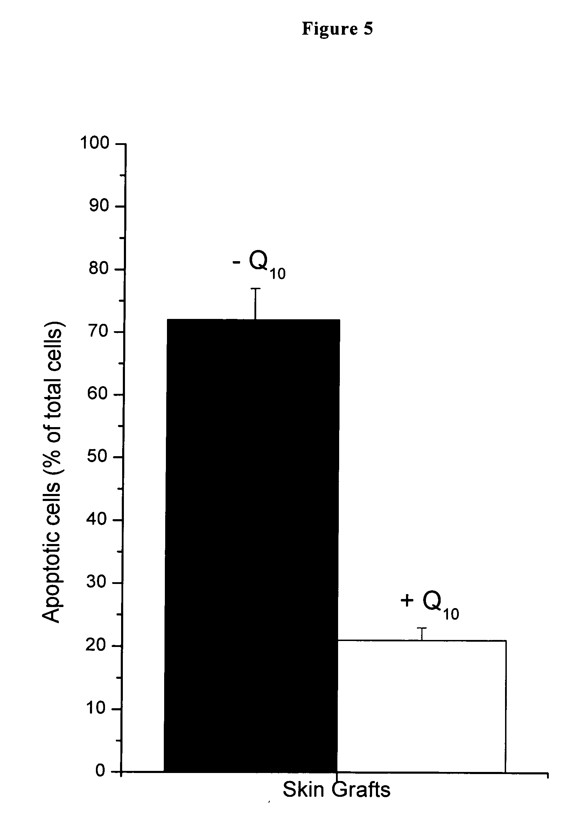

Hoechst Staining method confirmed in skin graft the same results obtained with adipocytes. The percentage of apoptotic cells was dramatically lower in glass slides of Q10 treated cells than in glass slides of non treated cells **(Figure 4).** Q10⁺ cells had the normal morphology of a living cell. Instead non treated cells presented nuclear condensation and fragmentation, tipical of an apoptotic tissue. Consequently, the number of apoptotic cells in skin grafts immersed in coenzyme Q10-saline solution was drastically decreased (**Figure 5**).

### REFERENCES

1. Tempestini A., Schiavone N., Papucci L., Witort E., Lapucci A., Cutri M., Donnini M. and Capaccioli S. "The mechanisms of apoptosis in biology and medicine: a new focus for ophthalmology" *Eur. J. Ophthalmol,* 13 Suppl 3: S11-18, 2003
2. Gastman B.R., Futrell W. and Manders E.K. "Apoptosis and Plastic Surgery" *Plast. Reconstr. Surg.* 111: 1481-1491, 2002
3. Brancato R., Schiavone N., Siano S., Lapucci A., Papucci L., Donnini M., Formigli L., Zecchi Orlandini S., Carella G., Carones F and Capaccioli S. "Prevention of corneal keratocyte apoptosis after argon fluoride excimer laser irradiation with the free radical scavenger ubiquinone Q10" *Eur. J. Ophthalmol 10: 32-38, 2000*
4. Brancato R., Fiore T., Papucci L., Schiavone N., Formigli L., Zecchi Orlandini S., Gobbi P.G., Carones F., Donnini M., Lapucci A., Capaccioli S. "Concomitant effect of topical application of ubiquinone Q10 and vitamin E to prevent keratocyte apoptosis after rabbit corneal excimer laser photoablation" *Journal of Refractive Surgery* 18:135-139, 2002
5. Papucci L., Schiavone N., Donnini M., Lapucci A., Witort E., Tempestini A., Formigli L, Zecchi Orlandini S., Orlandini G., Carella G., Brancato R. and Capaccioli S. "Coenzyme Q10 prevents apoptosis by directly inhibiting mitochondrial permeability transition independently of its free radical-scavenging property" *J. Biol*. *Chem.* 278:28220-28228, 2003
6. Coleman R.: "Facial recontouring with lipostructure" *Clinics in Plastics Surgery* 24:347-367, 1997
7. Rioux et al., *Journal of Lipid Research,* Vol. 43:167-173, 2002
8. Lehninger, A.L., Nelson, D.L., and Cox, M.M. *Principles of Biochemisrtry* 2_{nd} ed. New York: Worth Publishers, Inc., 480-483, 1993
9. Gonzales, D.H. and Neupert, W. "Biogenesis of mitochondrial c-type cytochromes" *J*. *Bioenerg. Biomembr.* 22: 753-768, 1990
10. Liu, X., Kim, C.N., Yang, J., Jemmerson, R., and Wang, X. "Induction of apoptotic program in cell-free extracts: requirement for dATP and cytochrome c" *Cell* 86: 147-157, 1996
11. Bernardi P. et al., Physiol. Rev. 79: 1127-1155 (1999).
12. Fontaine E. et al., Journal of Bioenergetics and Biomembranes, vol. 31 n. 4, 335-345 (1999).

## Claims

1. A method for preventing or inhibiting apoptosis in cells from explanted adipose or cutaneous human tissues, which comprises *ex vivo* contacting said tissues, or cells isolated therefrom, with coenzyme Q10.

2. A method according to claim 1, wherein the coenzyme Q10 is added to a culture of said tissues or cells.

3. A method according to claim 2, wherein coenzyme Q10 is diluted in a water solution containing an amphipathic co-solvent.

4. A method according to claim 1, wherein the explanted adipose or cutaneous human tissues or cells thereof are subjected to *ex vivo* treatments necessary to their subsequent implantation.

5. A method according to claim 4, wherein said tissues or cells are subjected to fractionation, separation, centrifugation and freezing.

6. The use of coenzyme Q10 for the preparation of a pharmaceutical composition for inhibiting apoptosis in cells of transplanted human tissues.

7. The use according to claim 6, for reducing the ischemia and reperfusion injury in transplanted tissues.

8. The use according to claims 6 or 7, wherein the transplanted human tissues are cutaneous and adipose tissues.

9. The use according to claims 6-8, wherein the pharmaceutical composition is suitable for topical administration.

10. The use according to claim 9, wherein the composition is in the form of solution, cream, ointment, gel or controlled-release transdermal delivery system.
